## Europäisches Patentamt
## European Patent Office
## Office européen des brevets

(11) Veröffentlichungsnummer: **0 213 581**
**A2**

# (12) EUROPÄISCHE PATENTANMELDUNG

(21) Anmeldenummer: **86111708.3**

(22) Anmeldetag: **23.08.86**

(51) Int. Cl.4: **C12P 21/00** , G01N 33/577 , A61K 39/395 , A61K 49/02 , A61K 43/00 , A61K 47/00

(30) Priorität: **02.09.85 DE 3531301**

(43) Veröffentlichungstag der Anmeldung: **11.03.87 Patentblatt 87/11**

(84) Benannte Vertragsstaaten: **AT BE CH DE FR GB IT LI LU NL SE**

(71) Anmelder: **BEHRINGWERKE Aktiengesellschaft Postfach 1140 D-3550 Marburg 1(DE)**

(72) Erfinder: **Bosslet, Klaus, Dr. Am Schlag 5 D-3550 Marburg(DE)**
Erfinder: **Lüben, Gerhard, Dr. An der Haustatt 25 D-3550 Marburg(DE)**
Erfinder: **Kanzy, Ernst-Jürgen Huteweg 3 D-3556 Weimar(DE)**
Erfinder: **Sedlacek, Hans-Harald, Dr. Sonnenhang 3 D-3550 Marburg(DE)**

(74) Vertreter: **Meyer-Dulheuer, Karl-Hermann, Dr. et al HOECHST Aktiengesellschaft Zentrale Patentabteilung Postfach 80 03 20 D-6230 Frankfurt/Main 80(DE)**

(54) **Monoklonale Antikörper gegen tumorassoziierte Glykoproteine, Verfahren zu ihrer Herstellung sowie ihre Verwendung.**

(57) Es werden der als MAK 436/15 bezeichnete monoklonale Antikörper vom IgG₃-Isotyp mit Kappa-Kette, der mit einem Glykoprotein (Antigen 1) reagiert, das ein Molekulargewicht (MW) von über 200 kDa unter nicht-reduzierenden Bedingungen hat, der als MAK 494/32 bezeichnete monoklonale Antikörper vom IgG-Isotyp mit Kappa-Kette, der mit einem Glykoprotein (Antigen 2) reagiert, das ein MW von über 200 kDa unter nicht-reduzierenden wie unter reduzierenden Bedingungen hat, und zwei als MAK 495/19 und 495/36 bezeichnete monoklonale Antikörper vom IgG₃-Isotyp mit Kappa-Kette, die mit einem Glykoprotein (Antigen 3) reagieren, das ein MW von über 200 kDa unter nicht-reduzierenden wie unter reduzierenden Bedingungen hat, sowie ein Verfahren zu ihrer Herstellung beschrieben. Diese Antikörper können zur Bindung an die entsprechenden Antigene auf diese Antigene tragenden Zellen und Geweben und deshalb als Diagnostika, Therapeutika oder als Träger für einen pharmazeutischen Wirkstoff verwendet werden.

## Monoklonale Antikörper gegen tumorassoziierte Glykoproteine, Verfahren zu ihrer Herstellung sowie ihre Verwendung

Die Erfindung betrifft monoklonale Antikörper (MAK), die an definierte zytoplasmatische, membranassoziierte oder sezernierte Antigene binden sowie ein Verfahren zu ihrer Herstellung. Diese Antikörper können als Diagnostikum, Wirkstoff oder Wirkstoffträger verwendet werden.

Verfahren zur Immunisierung mit definierten, isolierten Antigenen und zur Produktion von Antikörpern gegen solche Antigene sind bekannt. Es ist auch bekannt, mit ungereinigtem Antigenmaterial zu immunisieren und diejenigen Antikörper zu selektieren, die eine bestimmte Komponente einer solchen Antigenmischung erkennen.

Es ist uns gelungen, monoklonale Antikörper mit vorteilhaften Eigenschaften zu selektieren. Diese sind dadurch charakterisiert, daß sie mit bestimmten Epitopen auf Proteinantigenen reagieren:

MAK 436/15 reagiert mit einem Glykoprotein, das ein Molekulargewicht von über 200 kDa unter nicht-reduzierenden Bedingungen hat (Antigen 1). Das von diesem MAK erkannte Epitop ist gegen Formaldehydfixierung oder Paraffineinbettung ebenso wie gegen Neuraminidasebehandlung resistent. Perjodatoxidation zerstört jedoch dieses Epitop. MAK 436/15 gehört zum IgG₃-Isotyp mit einer Kappa-Kette als leichter Kette.

MAK 494/32 erkennt ein Epitop auf einem Glykoprotein (Antigen 2) von einem Molekulargewicht von über 200 kDa unter nicht-reduzierenden oder unter reduzierenden Bedingungen, welches sowohl resistent gegen Formaldehydfixierung oder Paraffineinbettung als auch gegen Neuraminidasebehandlung ist. Perjodatoxidation führt zur Zerstörung dieses Epitops. MAK 494/32 gehört zum IgG₁-Isotyp mit einer Kappa-Kette als leichter Kette.

MAK 495/19 oder MAK 495/36 erkennen ein Epitop auf einem Glykoprotein (Antigen 3) von einem Molekulargewicht von über 200 kDa unter nicht-reduzierenden oder unter reduzierenden Bedingungen, welches sowohl gegen Neuraminidasebehandlung als auch gegen Perjodatoxidation resistent ist. Im Gegensatz zu den Epitopen auf den Antigenen 1 und 2 ist dieses Epitop nicht resistent gegen Formaldehydfixierung oder Paraffineinbettung. MAK 495/19 und 495/36 sind vom IgG₃-Isotyp mit Kappa-Kette.

MAK 494/32 und MAK 495/36 sind in der Lage, die ADCC-Reaktion zu vermitteln (siehe Methodik: D. Herlyn et al. The Journal of Immunology 134, 2, 1300-1304 (1983).

Gegenstand der Erfindung ist ein als MAK 436/15 bezeichneter monoklonaler Antikörper vom IgG₃-Isotyp mit Kappa-Kette, der mit einem Glykoprotein (Antigen 1) reagiert, das ein Molekulargewicht (MW) von über 200 kDa unter nicht-reduzierenden Bedingungen hat, ein als MAK 494/32 bezeichneter monoklonaler Antikörper vom IgG₁-Isotyp mit Kappa-Kette, der mit einem Glykoprotein - (Antigen 2) reagiert, das ein MW von über 200 kDa unter nicht-reduzierenden wie unter reduzierenden Bedingungen hat, und zwei als MAK 495/19 und 495/36 bezeichnete monoklonale Antikörper vom IgG₃-Isotyp mit Kappa-Kette, die mit einem Glykoprotein (Antigen 3) reagieren, das ein MW von über 200 kDa unter nicht-reduzierenden wie unter reduzierenden Bedingungen hat.

MAK 436/15 erkennt ein Epitop auf Antigen 1 mit folgender Zugänglichkeit auf menschlichen Geweben, humanen Tumor-Xenotransplantaten auf der nackten Maus oder in vitro kultivierten Zell-Linien, wobei im Falle der Zell-Linien der Epitopnachweis mittels einer indirekten Immunfluoreszenzmethode auf lebenden Zellen (J. immunol. Methods 1977, 15, 57-66), im Falle des Xenotransplantates oder Humantumorgewebes mittels der indirekten Immunperoxidasemethode (J. clin. Path. 1979, 32, 971-978) erbracht wurde:

Die Colonkarzinomzell-Linie DE-TA und die Pankreaskarzinomzell-Linie PaTuI zeigen eine deutliche Membranfluoreszenz. Die Xenotransplantate der Pankreaskarzinome PaTuI und PaTuII und das kleinzellige Xenotransplantat eines Lungenkarzinomes B110 zeigen eine positive Immunfärbung in einzelnen Gewebearealen, wohingegen auf dem Lungenkarzinom BroCa17 und dem Pankreaskarzinomgewebe PaTuIII keine Reaktion nachzuweisen ist.

Auf menschlichen Karzinomen reagiert MAK 436/15 mit 8 hoch differenzierten Adenokarzinomen der Lunge von 55 getesteten anderen Lungenkarzinomen (sowohl sezernierte Produktanfärbung als auch Zytoplasma-und Membranfärbung). Einige Plattenepithel-Karzinome der Lunge reagieren ebenfalls. Des weiteren sind etwa 50% der Colonkarzinome und den hiervon abgeleiteten Lebermetastasen, etwa 60% der Pankreaskarzinome, etwa 40% der Ovarialkarzinome zu 10-20% der Tumorareale (starke Reaktion mit Sekret) und einige wenige Zellen bei 50% der Mammakarzinome reaktiv.

MAK 436/15 zeigt keine wesentliche Bindung an Lymphknoten, Tonsillen, periphere Blutleukozyten, normale Leber und normales Colon. Das dysplastische Epithel der Lunge, die inter-und intralobulären Gänge des Pankreas und einige wenige azinäre Zellen, sowie die Magendrüsen und einige wenige Zellen in der Milz zeigen eine Reaktion.

MAK 494/32 erkennt ein Epitop auf Antigen 2 mit folgender Zugänglichkeit auf menschlichen Geweben, humanen Tumorxenotransplantaten auf der nackten Maus oder in vitro kultivierten Zell-Linien, wobei die Bindung wie zuvor nachgewiesen wurde:

Die Colonkarzinomzell-Linie DE-TA zeigt eine deutliche Membranfluoreszenz. Die Xenotransplantate der Pankreastumore PaTuI und II, I.W. und 14 und die Xenotransplantate der Colonkarzinome 4 und 5 zeigen eine deutliche Zytoplasma-und Zellmembranreaktion.

Auf menschlichen Karzinomen reagiert MAK 494/32 mit etwa 80% der Grad I und II Adenokarzinome des Pankreas, wohingegen keine nennenswerten Reaktionen mit den Grad III Pankreaskarzinomen zu beobachten sind. Weiterhin werden etwa 70% der Colonkarzinomlebermetastasen und etwa 30% der Colonkarzinomprimärtumore immunhistologisch angefärbt. Einige wenige Tumorzellen bei etwa 40% der Lungenkarzinome sind reaktiv.

Normales Lungen-, Leber-, Brust-, Nieren-, Milz-, Lymphknoten-, Knochenmark-und Bindegewebe sowie Muskulatur und periphere Blutleukozyten zeigen keine nennenswerte Immunreaktionen mit MAK 494/32. Allerdings färben sich die mucusproduzierenden Zellen des Magens und des Colons (Becherzellen) sowie einige Zellen der äußeren Colonschleimhaut an. Im entzündeten Pankreas zeigt das Gangsystem eine Positivreaktion, wohingegen das exokrine und endokrine Pankreasgewebe nicht reagieren.

Das Molekulargewicht des durch MAK 494/32 definierten Antigens (über 200 kDa) unterscheidet sich von den durch die MAK AR2-20 und AR1-28 mit 190 kDa definierten Glykoproteinen (Cancer Res., 1985, 45, 1723-1729). Des weiteren ist die Epitopzugänglichkeit für MAK 494/32 derart, daß er präferentiell mit den hoch differenzierten Adenokarzinomen des Pankreas reagiert, wohingegen die MAK AR2-20 und AR1-28 mit allen getesteten Pankreaskarzinomen reagieren.

Deutliche Unterschiede zwischen MAK 494/32 und dem MAK DUPAN-2 (JNCI, 1985, 72, 999-1003) liegen in der Epitopzugänglichkeit (DUPAN-2 reagiert mit allen Pankreas-und Gallenblasenkarzinomen und vielen Drüsengeweben) und der Neuraminidase-Resistenz (DUPAN 2 erkennt ein neuraminidase-sensitives Epitop). Die in Cancer Res., 1985, 45, 1402-1407 beschriebenen MAK C54-0, C1-N3 und C1-P83 unterscheiden sich von MAK 494/32 in bezug auf Zugänglichkeit der Epitope und des definierten Antigens - (Molekulargewicht von 122 kDa für C54-0).

MAK 495/36 oder 495/19 erkennen ein Epitop auf Antigen 3 mit folgender Zugänglichkeit auf menschlichen Geweben, humanen Tumorxenografts auf der nackten Maus und in vitro kultivierten Zell-Linien, wobei eine Reaktion wie zuvor nachgewiesen wurde:

Die Colonkarzinomzell-Linie DE-TA zeigt eine deutliche Membranfluoreszenz. Alle getesteten Humantumorxenotransplantate wie die Bronchialkarzinome BroCa-11, GOT-1, MR-21E560Sp, BroCa-33, BroCa-53, B98, die Pankreaskarzinome PaTuI, PaTuII und die Colonkarzinome Ca 4,5, Le-Met Co. Ca 2, DE-TA, Le-Met Co. Ca 9 und Le-Met Co. Ca 3 zeigen eine starke zytoplasmatische und membranöse Reaktion.

Auf menschlichen Lungenkarzinomgeweben zeigt MAK 495/36 eine deutliche Membranreaktion mit etwa 100% der getesteten Fälle unterschiedlicher Histologie. Des weiteren zeigen etwa 90% der Pankreaskarzinome, Colonkarzinom-Lebermetastasen, Ovarial-und Blasenkarzinome sowie Mammakarzinome eine starke membranöse Reaktion. Keine immunhistologische Färbung ist bei peripheren Blutleukozyten, Lymphozyten, Milz, Blutgefäßen, Bindegewebe und Muskulatur zu beobachten. Beim normalen Colon reagieren die Schleimhaut und die Krypten positiv, in der Leber das Gallengangepithel und im Magen das Drüsengewebe. Das Epithel der Lunge und das exo-und endokrine Pankreasgewebe zeigen eine starke Reaktion.

Die für die Erzeugung obiger monoklonaler Antikörper benutzte Methodik ist in der Deutschen Offenlegungsschrift 33 29 184 auf den Seiten 3 bis 5 beschrieben.

Gegenstand der Erfindung ist weiterhin ein Verfahren zur Herstellung eines der oben beschriebenen monoklonalen Antikörper, dadurch gekennzeichnet, daß ein Säugetier mit Zellen der Lungenkarzinomzell-Linie MR22E572 oder der Colonkarzinomzell-Linie DE-TA immunisiert, Milzzellen aus einem solchen immunisierten Tier entnommen, mit der Zell-Linie X63 Ag8.653 fusioniert, die Hybridome selektiert und der MAK gewonnen werden.

Als Immunogen zur Induktion des MAK 436/15 dient die Zell-Linie MR22E572 und zur Induktion der MAK 494/32, 495/36 und 495/19 die DE-TA Zell-Linie. Säugetiere, vorzugsweise Mäuse, werden mit Zellen einer dieser Zell-Linien immunisiert und die Milzzellen aus solchen Tieren mit der X63 Ag8.653 Zell-Linie fusioniert.

Die entstehenden Hybridome werden darauf getestet, ob sie Antikörper der gewünschten Spezifität enthalten. Unter den ausgetesteten Hybridomüberständen befinden sich einige, die Antikörper der oben beschriebenen Spezifitäten enthalten. Die diese Antikörper sezernierenden Hybridome werden kloniert und die von diesen Hybridomaklonen gewonnenen monoklonalen Antikörper benutzt, um das von ihnen erkannte Antigen immunchemisch zu charakterisieren. Das Molekulargewicht wird bestimmt im Vergleich zu kommerziell erhältlichen Markern. Die Antigene können affinitäts-chromatographisch gereinigt werden.

Zusätzlich wird die Bindung der monoklonalen Antikörper an histologische Präparate mittels der indirekten Immunperoxidasetechnik gemessen (J. clin. Pathol., 1979, 32, 971).

Weiterhin wird die molekulare Charakterisierung der von den monoklonalen Antikörpern erkannten Antigene oder Antigenmolekülsubpopulationen zusätzlich zur Westernblot-Analyse mittels Radioimmunpräzipitation (Behring Institute Mitteilungen, 1984, 74, 27-34) durchgeführt.

Aufgrund ihrer Reaktivität mit verschiedenen Epitopen auf menschlichen Geweben können die MAK 436/15, 494/32 und 495/36 oder 495/19 zur immunhistologischen Differenzierung zwischen epitop-positiven und epitop-negativen Geweben oder Körperflüssigkeiten benutzt werden. Die oben beschriebenen MAK sind des weiteren in der Lage, sich in radioaktiv markierter Form an Tumore in vivo zu binden (436/15, 494/32) und mit ihrer Hilfe können frühe Metastasen in drainierenden Lymphknoten immunszintigraphisch nachgewiesen werden (495/36, 495/19).

Zusätzlich können diese monoklonalen Antikörper als Wirkstoffträger eingesetzt und zur Therapie maligner Erkrankungen benutzt werden. Weitere Anwendungsmöglichkeiten stellen die Hemmung der Tumorzellmetastasierung nach in vivo-Applikation oder die Bindung der monoklonalen Antikörper an antigen-tragende, metastasierende Tumorzellen dar. Außerdem können diese monoklonalen Antikörper ohne Mitwirkung anderer Toxine für die antigen-tragenden Tumorzellen toxisch sein oder nach Bindung an die Tumorzellmembran zu Differenzierungsvorgängen führen, die bösartige Tumorzellen zu benignen Zellen werden lassen.

Des weiteren können die die ADCC vermittelnden MAK 494/32 und 495/36 in nicht radioaktiv markierter Form nach systemischer oder regionaler Applikation zur Tumortherapie epitop-positiver Tumore eingesetzt werden.

Die beschriebenen monoklonalen Antikörper können aber auch in markierter Form als Diagnostika oder für die Analyse von Geweben und Körperflüssigkeiten und für die Radioimmunszintigraphie oder als Therapeutika für die Radioimmuntherapie oder für die Chemoimmuntherapie gebraucht werden.

Für diagnostische Zwecke werden die monoklonalen Antikörper je nach Test in einer Konzentration von 0,001 µg/ml bis 100 µg/ml eingesetzt.

Therapeutische Effekte werden in Mengen von 200 µg-100 mg/kg Körpergewicht erreicht.

Neben den intakten erfindungsgemäßen Antikörpern können auch ihre durch Enzyme (im besonderen Papain, Plasmin oder Pepsin) nach der dem Fachmann bekannten Verfahren hergestellten Spaltprodukte wie beispielsweise $F(ab')_2$ oder Fab Verwendung finden.

## Ansprüche

1. Als MAK 436/15 bezeichneter monoklonaler Antikörper vom $IgG_3$-Isotyp mit Kappa-Kette, der mit einem Glykoprotein (Antigen 1) reagiert, das ein Molekulargewicht (MW) von über 200 kDa unter nicht-reduzierenden Bedingungen hat, ein als MAK 494/32 bezeichneter monoklonaler Antikörper vom $IgG_1$-Isotyp mit Kappa-Kette, der mit einem Glykoprotein (Antigen 2) reagiert, das ein MW von über 200 kDa unter nicht-reduzierenden wie unter reduzierenden Bedingungen hat, und zwei als MAK 495/19 und 495/36 bezeichnete monoklonale Antikörper vom $IgG_3$-Isotyp mit Kappa-Kette, die mit einem Glykoprotein (Antigen 3) reagieren, das ein MW von über 200 kDa unter nicht reduzierenden wie unter reduzierenden Bedingungen hat.

2. Verfahren zur Herstellung des als MAK 436/15 bezeichneten monoklonalen Antikörper vom $IgG_3$-Isotyp mit Kappa-Kette, der mit einem Glykoprotein (Antigen 1) reagiert, das ein Molekulargewicht (MW) von über 200 kDa unter nicht-reduzierenden Bedingungen hat, des als MAK 494/32 bezeichneten monoklonalen Antikörper vom $IgG_1$-Isotyp mit Kappa-Kette, der mit einem Glykoprotein (Antigen 2) reagiert, das ein MW von über 200 kDa unter nicht-reduzierenden wie unter reduzierenden Bedingungen hat, des als MAK 495/19 oder des als 495/36 bezeichneten monoklonalen Antikörpers, beide vom $IgG_3$-Isotyp mit Kappa-Kette, die beide mit einem Glykoprotein (Antigen 3) reagieren, das ein MW von über 200 kDa unter nicht reduzierenden wie unter reduzierenden Bedingungen hat, dadurch gekennzeichnet, daß ein Säugetier mit Zellen der Lungenkarzinomzell-Linie MR22E572 oder der Colonkar zinomzell-Linie DE-TA immunisiert, Milzzellen aus einem solchen immunisierten Tier entnommen, mit der Zell-Linie X63 Ag8.653 fusioniert, die Hybridome selektioniert und der MAK gewonnen werden.

3. Verfahren nach Anspruch 2, dadurch gekennzeichnet, daß das Säugetier die Maus ist.

4. Verwendung eines monoklonalen Antikörpers nach Anspruch 1 in einem in vitro-Diagnostikum.

5. Verwendung eines monoklonalen Antikörpers nach Anspruch 1 als Träger für einen pharmazeutischen Wirkstoff.

6. Verwendung eines monoklonalen Antikörpers nach Anspruch 1 als Therapeutikum.

7. Verwendung eines monoklonalen Antikörpers nach Anspruch 1 in markierter Form als Diagnostikum oder für die Analyse von Geweben und Körperflüssigkeiten und für die Radioimmunszintigraphie.

8. Verwendung eines monoklonalen Antikörpers nach Anspruch 1 in markierter Form als Therapeutikum für die Radioimmuntherapie oder für die Chemoimmuntherapie.